# EUROPEAN PATENT APPLICATION

(11) **EP 0 870 823 A1**
(43) Date of publication of application: **14.10.1998**
(21) Application number: 98103344.2
(22) Date of filing: 26.02.1998
(51) Int. Cl.: C12M 1/00, C12M 1/34

(54) **System for monitoring the metabolic activity of living cells**

(30) Priority: 07.03.1997 IT TO970188
(71) Applicant: ISTITUTO TRENTINO DI CULTURA, I-38100 Trento (IT); Omega S.r.l., 16131 Genova (IT)
(72) Inventor: Zen, Mario, 38100 Trento (IT); Margesin, Benno, 38050 Povo (Trento) (IT); Lui, Alberto, 38100 Trento (IT); Chiarugi, Sergio, 16131 Genova (IT); Grattarola, Massimo, 16131 Genova (IT); Martinoia, Sergio, 16131 Genova (IT); Chiarugi, Luca, 16158 Genova Voltri (Genova) (IT)
(74) Representative: Gerbino, Angelo

(57) **Abstract**

The system for monitoring the metabolic activity of living cells (26) comprises: a cell (20) able to receive the cell population (26) to be monitored; means for supplying to a culture medium the cell (20), and means for detecting the pH value determined by the catabolism of the cell population (26) in the cell (20). This latter preferably comprises a casing which encloses a membrane (22) on which the cell population (26) can be fixed. In the casing there is formed a channel (30) for the culture medium and a channel (28) for supplying a solution containing the cell population (26). The channels (28, 30) flow alongside the membrane (22) on opposite sides and are separated from one another.

## Description

The present invention relates to a system for monitoring the metabolic activity of living cells, and to its operating procedure.

The object of the present invention is that of providing a system for monitoring the catabolic activity of eukaryote and/or prokaryote living cells, adapted to operate both continuously and intermittently, capable of effecting measurements both of qualitative and quantitative type and having a wide spectrum of applications which extend from research activity to routine analysis.

This object is achieved by a monitoring system having the characteristics specifically set out in the following claims.

Further advantages and characteristics of the present invention will become apparent from the following description given by way of non-limitative example, with reference to the attached drawings, in which:
Figure 1 is a schematic representation of a system according to the invention;
Figure 2 is an exploded representation of a component of the system of Figure 1 in one operating phase;
Figure 3 is an exploded representation of the component of Figure 2 in another operating phase;
Figure 4 is an exploded representation of an alternative embodiment of the component of Figures 2 and 3 in one operating phase;
Figure 5 is an exploded representation of the component of Figure 4 in another operating phase; and
Figure 6 is a representation of another component of the system of Figure 1.

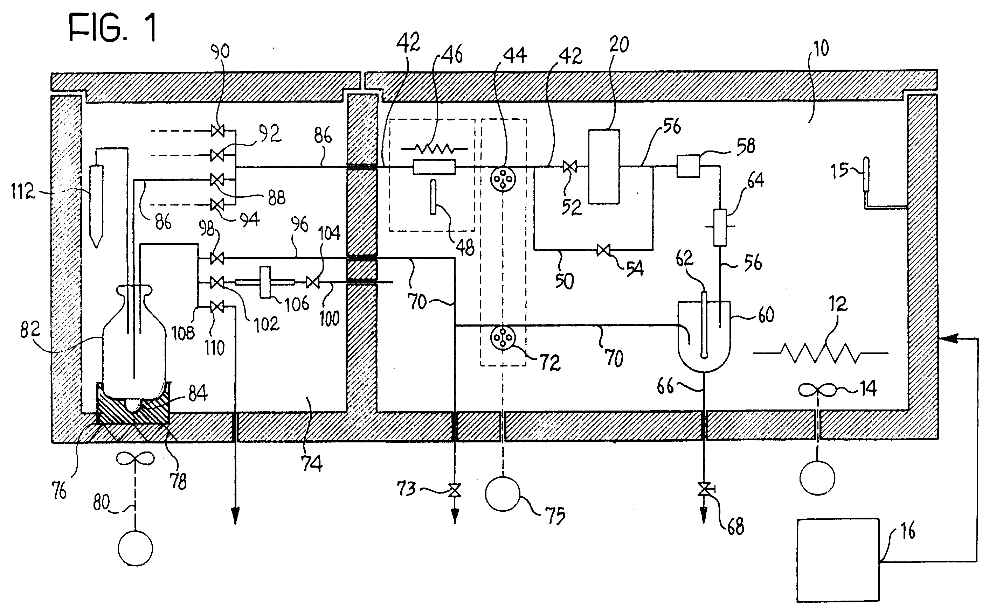

A system for monitoring the metabolic activity of eukaryote and/or prokaryote cells comprises (Figure 1) a measurement chamber 10 provided with temperature control means. These latter in particular comprise a heating resistor 12 with which are associated a motor driven fan 14 and a temperature transducer 15, and are governed in a manner known per se by an electronic central control unit 16 for control, acquisition and presentation of data.

In the chamber 10 is located a cell 20 in which the population of living cells to be monitored is present.

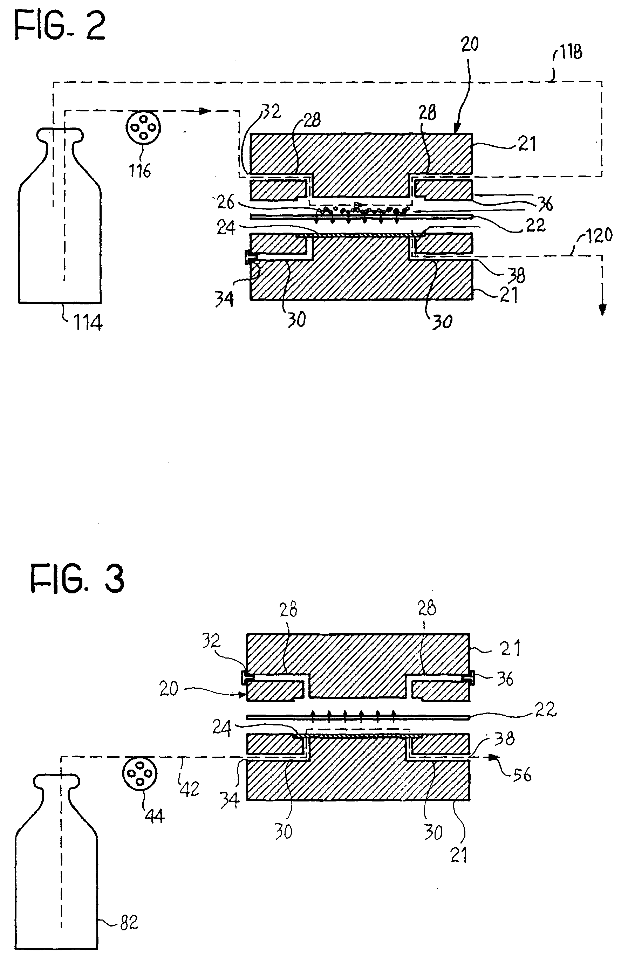

The cell 20 comprises (Figures 2 and 3) a casing formed by two shell-like assemblies 21 which enclose a membrane 22 and a porous support 24 for this (it is to be noted that, for greater clarity, Figures 2 and 3 show the shells 20 as separated). The membrane 22 constitutes an anchoring surface for the population of cells 26 to be monitored.

A channel 28 is formed in the casing for supply of a solution containing the cells 26 and a channel 30 for passage of a culture medium, the manner of operation of which will be described hereinafter. The channels 28, 30, provided with respective inlet and outlet apertures 32, 34; 36, 38 pass alongside the membrane 22 on opposite sides and are separated from one another.

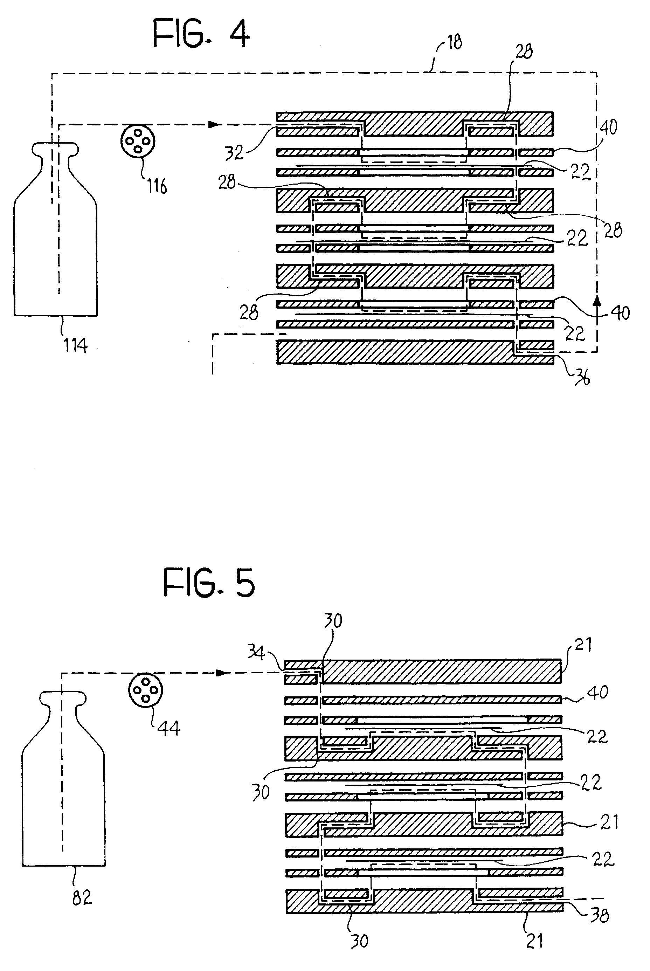

Alternatively it is possible to utilise a multiple cell (Figures 4 and 5) comprising a plurality of single cells of the type described above in which the channels 30 for passage of the culture medium are connected in series with one another as also, independently of the culture medium channels 30, are the channels 28 for the supply of the solution containing the cells 26. For simplicity of representation, in Figure 4 only the channels 28 for supply of the solution containing the cell population have been shown, whilst in Figure 5 only the channels 30 for passage of the culture medium are shown. Between the shells 21 there are further disposed seals 40 of silicone rubber or other material.

According to a further alternative embodiment not illustrated in the drawings, it is possible to utilise a cell constituted by a standard syringe filter of porosity suitable to retain the population of cells and formed of biocompatible material. Such filters are known per se and commonly utilised in laboratory practice.

However it is formed the cell 20 has (Figure 1) a duct 42 opening into it for supply of culture medium, in which are disposed a pump 44 and temperature control means comprising a further heating resistor 46 and a temperature transducer 48 also controlled by the central control unit 16.

In parallel with the cell 20 there is disposed a bypass duct 50. Respective switching valves 52, 54 are disposed upstream of the cell 20 and in the bypass duct 50.

From the cell 20 extends an exit duct 56 along which are located means for detecting the pH value determined by the catabolism of the cell population 26. Alternatively, in an embodiment not illustrated, these detection means can be integrated with the cell 20.

The detection means comprise an ISFET sensor 58 (Ion Sensitive Field Effect Transistor) of a type known per se. This sensor is capable of detecting the concentration of hydrogen ions in solution thanks to the presence of specific sites for the H⁺ and OH⁻ ions on the exposed surface of this solution, and is able to produce an electrical signal, in particular a voltage, depending on this concentration. The typical sensitivity at ambient temperature is about 50 mV/unit of pH.

Along the exit duct 56 is disposed a reservoir 60 containing a reference and/or working electrode 62 operable to polarise the solution to guarantee the operation of the sensor 58.

Also along the exit duct 56, between the sensor 58 and the reservoir 60, is located an air bubble detector 64. This latter can be of any commercially available type, for example optical or conductivity sensitive. Both the bubble detector 64 and the sensor 58 are connected to the central control unit 16.

The reservoir 60 is provided with a drain duct 66 with switching valves 68 and an overflow duct 70 in which are disposed a discharge pump 72 and a valve 73. Advantageously the pumps 72 and 44 constitute two different sections of the same peristaltic pump driven by a stepping motor 75.

Adjacent the measurement chamber 10 is an auxiliary chamber 74 provided with temperature control means. These latter in particular include a heat pump of the Peltier battery type 76 controlled by the central control unit 16. The heat extracted from the chamber 74 is dissipated to the environment via a finned heat dissipater 78 cooled by a motor driven fan 80.

In the chamber 74 there is contained a plurality of culture medium containers 82, for example four in number, of which, for simplicity, only one is shown in Figure 1.

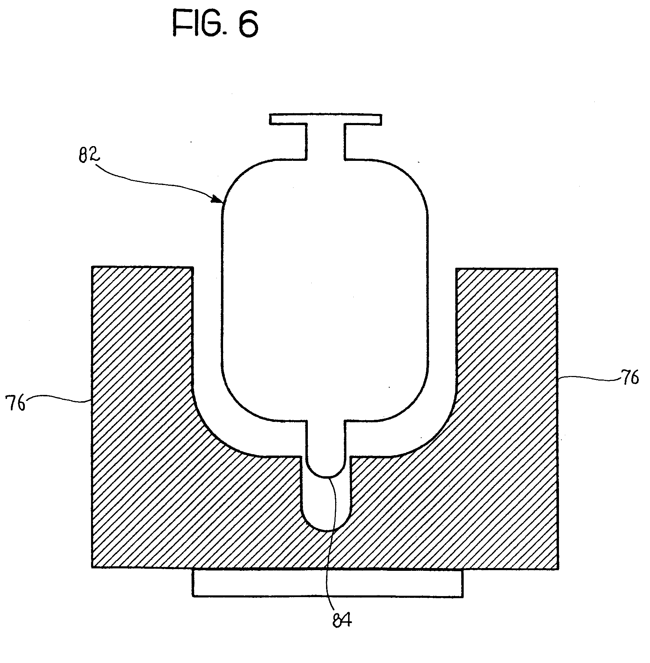

This latter is shaped substantially in the form of a bottle and is provided at its bottom with an oblong protuberance 84 (Figure 6).

The container 82 has (Figure 1) an outlet duct 86 which is provided with an interception valve 88 and is connected to the supply duct 42 leading to the cell 20. The diagram of Figure 1 shows the switching valves 90, 92, 94 disposed on the end section of the outlet ducts of the other three containers not illustrated, which ducts are all connected to the supply duct 42.

The container 82 has moreover an inlet duct 96 - which is provided with an interception valve 98 and is connected to the discharge duct 70 from the reservoir 60 - and a pressure balancing duct 100 in which there is disposed a filter 106 between two valves 102, 104.

From the container 82 extends a further overflow discharge duct 108 provided with a valve 110.

It will be understood that all the containers 82 are provided with ducts 96, 100 and 108 with associated valves and accessories although, for simplicity, Figure 1 shows only one container 82.

In the auxiliary chamber 74 there are further disposed means 112 for injecting desired additives into the containers 82. These injection means 112 comprise replaceable syringes of different capacities which can be operated by a stepping motor under the control of the central control unit 16.

A first mode of operation of the system described above for open circuit measurement of the base metabolism of a given cell population 26 is as follows.

First it is necessary to fix the cell population 26 to be monitored on to the membrane 22 of the cell 20 extracted from the chamber 10. For this purpose (Figure 2) the inlet aperture 34 of the channel 30 is closed and the apertures 32, 36 of the channel 28 are connected to a circulation circuit for a solution containing the cell population comprising a supply reservoir 114, a pump 116 and connection tubes 118.

After the flow of the solution along the membrane 22 of the cell 20 part of the cell population 26 is captured on the pores of the membrane 22 whilst part of the solvent percolates through these latter and is discharged through the outlet aperture 38 of the channel 30 downstream of which there is provided a discharge duct 120 having a lower pressure drop than that of the recirculation tubes 118 of the circuit.

In this way there is obtained a progressive concentration of the solution and the fixation on the membrane 22 of the major part of the cell population 26. These cells can thus be subjected subsequently to measurements even though they were originally present in solution at a dilution level so high as to render such an analytical procedure substantially impracticable. The possibility of effecting a concentration of the sample therefore constitutes one of the most significant advantages of the monitoring system of the present invention.

The operation of the multiple cell is substantially similar (Figure 4) with the difference that the solution which exits from one cell is delivered to subsequent cells until reaching the last cell from where it is recycled.

As appears from Figures 2 and 4, the solution flows parallel to the membrane or membranes 22. If on the other hand a cell of standard filter type - to which reference has been made above - is used there is the disadvantage of a progressive restriction due to the flow through the filter.

The cell 20 charged with the cell population 26 to be monitored is then located within the measurement chamber 10 closing the apertures 32, 36 of the channel 28 and connecting the apertures 34, 38 of the channel 30 respectively to the supply duct 42 and to the outflow conduit 56 (Figures 1 and 3).

Then, once a liquid culture medium having good nutritional characteristics for the cell population 26 has been introduced into the container 82 the valves 88, 102, 104, 52 and 73 are opened keeping the remaining valves 90, 92, 94, 98, 110, 68 and 54 closed and starting the pumps 44 and 72 to effect a washing phase of the components of the system and to bring them to the desired temperature.

In this way the culture medium flows through the outlet duct 86, the valve 88, the supply duct 42, being preheated thanks to the resistance 46, the valve 52, the cell 20, the sensor 58, the detector 64 and the reservoir 60, from which it is discharged through the duct 70 and the valve 73. At the same time the opening of the valves 102, 104 allows filtered air to enter the container 82 so as to re-establish the pressure balance which otherwise would fall following the outflow of culture medium.

The operating parameters, such as temperature of the chambers 10, 74, rate of flow, and duration of the various operations, are controlled by the central control unit 16 to which possible alarm signals can be sent by the detector 64 in the case of the presence of air bubbles over a protracted time.

In particular it is convenient to adjust the temperature of the chamber 74 to rather low temperatures, of the order of 10°C, so as to prevent bacteria which may have infiltrated in from the outside, from being able to develop and multiply. The resistance 46 can then heat the culture medium arriving from the chamber 74 to a higher temperature, of the order of 37°C, favourable for the development of the cell population 26 to be monitored.

If necessary the above-described washing phase can be repeated once or twice.

Then a blank phase is performed, in which, as opposed to the washing phase, the valve 52 is closed whilst the valve 54 is open. In this way the culture medium flows through the bypass duct 50 without coming into contact with the cell 20 and the cell population 26 contained in it.

In this way, once the pumps 44, 72, and consequently the flow of liquid in the outflow duct 56 is stopped, the pH values detected by the sensor 58 correspond to those of the virgin culture medium and can serve as a comparison term (reference) in the subsequent measurement phase.

In this latter phase the condition of the valves is the same as that of the washing phase, but without a flow in the bypass duct 50. The activation of the pumps 44, 72 therefore causes a flow of culture medium through the cell 20 and in particular along the channel 30 (compare Figure 3 for the single cell and Figure 5 for the multiple cell). Through the membrane or membranes 22 the culture medium can thus yield nutritive principles to the cell population 26 and absorb the H⁺ ions produced by the cellular catabolism. After a predetermined time pumps 44, 72 are stopped and a pH measurement of the medium is taken with the ISFET sensor 58 of the medium loaded with the products of catabolism which flow out through the outflow duct 56. This value is transmitted to the central control unit 16.

The above-described measurement phase can be repeated any desired number of times so as to monitor the evolution in time of the pH value. The detected values can be displayed, for example in the form of a graph of pH as a function of time, under the control of the central control unit 16.

The rate of flow of culture medium through the cell 20 in each measurement phase can be established at will. In particular, this rate of flow can vary from a minimum value which allows during each phase replacement of only a micro quantity of the culture medium present in the cell 20, to a maximum value which allows complete replacement in one phase of the quantity of culture medium present in the cell 20.

In the first case the cell population 26 is in an environment with variable physiological conditions, in the second case they are constant.

According to the concentration, type and activity of the cell population 26 to be monitored it is possible to choose the most suitable value for the rate of flow. In particular, if the concentration is low it is preferable to choose a low flow rate so as to increase the sensitivity of the measurement. If on the other hand the concentration is high, it is preferable to choose a high flow rate so as to avoid variations over time of the physiological conditions which would induce significant variations in the response.

The central control unit 16 can moreover be programmed to command the performance, at a predetermined frequency/rate, of a blank phase as described above to compensate for possible drift of the sensor 58 and to test the state of the pH of the virgin culture medium.

The possible presence of air bubbles in the outflow duct 56 is indicated by the detector 64 to the central control unit 16 which acts to interrupt the measurement phase and to effect, if necessary, one or more further washing phases.

The measurement of the base metabolism of a given cell population 26 can also be effected in a closed cycle rather than an open cycle manner.

In this case washing and blank phases are performed similar to what has been described above.

In the performance of the measurement phase, on the other hand, the culture medium is withdrawn from a container 82 different from that used for the other phases and filled only in correspondence with the protuberance 84. The oblong shape of this latter ensures, however, a certain head even when operating with reduced quantities of culture medium, and thus avoids the formation of air bubbles when this latter is put into circulation.

To this end the pumps 44, 72 are activated, keeping the valve (for example number 88) associated with this latter container open and the valve 90 associated with the container utilised for the washing and blank phases closed. A further difference with respect to the open circuit measurement phase lies in the fact that the discharge valve 73 is now closed, whilst the valve 98 is open. In this way the culture medium flowing in the discharge duct 70 is recycled to the container 82 through the duct 96 and the valve 98 rather than being discharged to the outside.

In this case it is also possible to effect blank cycles at a predetermined frequency, in which the virgin culture medium is supplied from the container associated with the valve 90.

With this mode of operation, advantageously in the presence of very low cell concentrations, the same culture medium can be subjected several times to the action of the cell population as long as its pH value remains sufficiently high to allow the cells to remain alive.

For example, it is possible to know the quantity of cells present in a sample to be examined by performing a measurement phase with corresponding parameters for this sample and for a known standard and comparing the number of phases necessary to reach the same acidity value respectively.

The system of the invention also makes it possible to measure the variation of the basal metabolism of a cell population induced by an external agent.

In this case one can first operate in a similar manner to the open cycle case as previously described, obtaining a relative indication of the basal metabolism in the presence of a culture medium without additives.

Then measurement phases are performed by supplying the cell 20 with culture medium having a desired external agent added to it, from a different container 82 (by opening the valve disposed on the associated outlet duct 86 and closing the valve associated with the preceding container).

In this case it is also possible to perform blank phases at a predetermined frequency, in which the virgin culture medium is supplied from the container containing culture medium without additives.

The external agent added to the culture medium induces variations in the metabolic activity of the cell population with respect to the culture medium without additives. From a comparison of the measurements made with and without additives it will therefore be possible to arrive at a measurement of the effects of the additives themselves on a cell population. This manner of operation finds significant applications in the immunological, pharmacological and cosmetics fields and in many cases can advantageously replace *in vivo* tests.

Measurement of the variation of the basal metabolism of a cell population induced by an external agent can also be made in a closed cycle.

This manner of operation is identical to the preceding open cycle process with the difference that in the measurement phases the valve 73 is closed and the valve 98 is open.

The culture medium to which an external agent has been added is therefore drawn from one of the containers 82, flows through the cell 20 and is recycled to the same container 82 maintaining the discharge valve 73 closed.

The same culture medium is thus in contact with the cell population 26 several times so that, at the same concentration of this latter, the response of the system is more rapid than in an open cycle.

The closed cycle manner of operation moreover makes it possible to monitor cell populations having very low concentrations which cannot be analysed in the open cycle mode, however having the disadvantage of producing a progressive acidification of the culture medium which, once exceeding a certain value, ends by causing the death of the cell population.

A further mode of operation of the system envisages a function similar to that of open cycle measurement of the basal metabolism, with the difference that the measurement phases are performed in a predetermined succession with different culture media supplied from time to time from different containers 82.

This mode of operation is advantageously employed when composite cell populations have to be monitored. In fact the metabolisms of cells belonging to different families (stock) have different responses depending on the type of culture medium utilised, which makes it possible to encourage or inhibit the metabolism of cells from a specific family with respect to that of cells from other families.

It is thus possible to verify the effective presence of a family present in a mixture with others in a sample to be analysed.

Specific examples of the application of this mode of operation are for example the verification of the presence of somatic cells in milk (essential for the determination of the bacteriological load) or the verification of the presence in food of salmonella, which is usually associated with other cell families.

The system of the invention also allows easy disinfection and sterilisation between one test and another, whereby to avoid the residues of culture medium or cell populations of preceding tests from influencing the results of subsequent tests.

To this end it is possible, when desired, to perform a disinfection programme by filling all the containers 82 with a biocide solution, for example hypochlorite, dismantling the cell 20 with the direct connection of the ducts 42, 56, opening the valves 88, 90, 92, 94, 98, 52, 54, 110, closing the valve 73 and operating the pumps 44, 72.

In this way all the components of the system through which culture medium has passed during the performance of the measurement, are in contact with the biocide solution and are sterilised. The discharge duct 108, in which the valve 110 is open, acts as an overflow and allows the elimination of excess biocide solution from the associated container 82.

After the disinfection programme a rinsing programme is performed in a similar manner to the preceding one and utilising distilled water, possibly sterile, as the treatment fluid. In this way possible residues of biocide solution are eliminated and the system is then ready for subsequent measurement procedures.

From what has been explained above the flexibility of the system of the invention is evident, which can be equally well utilised for a wide variety of different fields: for example pharmacological, immunological, biological, biomedical, for production tests, for testing water, cosmetics, toxicological, ecological, bacteriological tests, tests on food, industrial waste, drinking water and the like.

Naturally it is understood that the principle of the invention remaining the same, the details of construction and the embodiments can be widely varied with respect to what has been described above, without by this departing from the ambit of the present invention.

## Claims

1. A system for monitoring the metabolic activity of living cells (26), characterised in that it comprises:
- a cell (20) which can receive a cell population (26) to be monitored,
- means for supplying a culture medium to the cell (20), and
- means for detecting the pH value caused by catabolism of the cells (26) in the said cell (20).

2. A system according to Claim 1, characterised in that the said detection means comprise a sensor (58) operable to produce an electrical signal correlated to the detected pH value.

3. A system according to Claim 2, characterised in that the said sensor (58) is disposed in an outflow duct (56) from the cell (20) or is integrated with the cell (20).

4. A system according to Claim 2 or Claim 3, characterised in that the said sensor (58) is of ISFET type.

5. A system according to Claim 4, characterised in that along the said outflow duct (56) there is disposed a reservoir (60) containing a reference electrode and/or working electrode (62) operable to polarise the solution in order to ensure operation of the sensor (58).

6. A system according to Claim 5, characterised in that an air bubble detector (64) is located along the said outflow duct (56), between the said sensor (58) and the said reference electrode (62).

7. A system according to Claim 5 or Claim 6, characterised in that the said reservoir (60) is provided with a drain duct (66) and an overflow duct (70), in which are disposed an outflow pump (72) and a discharge valve (73).

8. A system according to any preceding claim, characterised in that a bypass duct (50) is disposed in parallel with the said cell (20), respective interception valves (54, 52) being disposed on the said bypass duct (50) and upstream of the cell (20).

9. A system according to any preceding claim, characterised in that the said cell (20) comprises a casing which encloses a membrane (22) on which the said cell population (26) can be fixed, the said casing having a channel (30) formed therein for passage of the said culture medium, and a channel (28) for supply of a solution containing the cell population (26), the said channels (28, 30) passing alongside the membrane (22) on opposite sides thereof and being separated from one another.

10. A system according to Claim 9, characterised in that it comprises a plurality of cells the culture medium channels (30) of which are connected in series with one another as also, independently of the culture medium channels (30), are the channels (28) for supply of solution containing the cell population (26).

11. A system according to any preceding claim, characterised in that the said cell (20) is located in a measurement chamber (10) provided with temperature control means.

12. A system according to any preceding claim, characterised in that the said means for supplying culture medium to the cell (20) comprise a supply duct (42) along which is disposed a pump (44), and temperature control means for the said duct (42).

13. A system according to any preceding claim, characterised in that it includes an auxiliary chamber (74) containing at least one container (82) for the said culture medium and provided with temperature control means (112) for injecting desired additives into the said container (82), the said container (82) having an outlet duct (86) which is provided with interception valves (88, 90, 92, 94) and is connected to the supply duct (42) of the cell (20), an inlet duct (96) which is provided with an interception valve (98) and is connected to the discharge duct (70) of the reservoir (60), and a pressure balancing duct (100) in which there is disposed a filter (106).

14. A system according to Claim 13, characterised in that the said at least one container (82) is shaped substantially in the form of a bottle and is provided with an oblong protuberance (84) at its bottom.

15. A system according to Claim 14, characterised in that it comprises a plurality of containers (82).

16. A process for monitoring the metabolic activity of cell populations (26), which provides for the use of a system according to any preceding claim.
